# EUROPEAN PATENT APPLICATION

(11) **EP 2 930 497 A1**
(43) Date of publication of application: **14.10.2015**
(21) Application number: 14305509.3
(22) Date of filing: 07.04.2014
(51) Int. Cl.: G01N 21/76, G01N 33/58

(54) **Enzyme-independent photon emission**

(71) Applicant: INSTITUT PASTEUR, 75015 Paris (FR)
(72) Inventor: Dragavon, Joseph, Boulder, CO 80301 (US); Holland, Alexandra, Boulder, CO 80301 (US); Rekiki, Abdessalem, 94000 Creteil (FR); Debarbieux, Laurent, 92290 CHATENAY-MALABRY (FR); Tournebize, Régis, 94800 VILLEJUIF (FR); Shorte, Spencer, 28130 SAINT-PIAT (FR)
(74) Representative: Leblois-Préhaud, Hélène Marthe Georgette

(57) **Abstract**

The invention relates to a new method for generating a luminescent signal, which is based on an enzyme-independent photon emission mechanism (EiPE), whereby luminescent light is generated when a bioluminescent substrate interact with a physical surface in the absence of any catalytic enzyme. In particular embodiments, the method is used to stimulate light emission by fluorescent molecules. The invention relates also to kits for implementing a method according to the present invention and to the use of a bioluminescent substrate, in the presence of a physical surface, to produce a luminescent signal in the absence of enzymatic catalyst.

## Description

The invention relates to a new method for generating a luminescent signal, which is based on an enzyme-independent photon emission mechanism (EiPE), whereby luminescent light is generated when a bioluminescent substrate interacts with a physical surface in the absence of any catalytic enzyme. In particular embodiments, the method is used to stimulate light emission by fluorescent molecules. The invention relates also to kits for implementing a method according to the present invention and to the use of a bioluminescent substrate, in the presence of a physical surface, to produce a luminescent signal in the absence of enzymatic catalyst.

Luminescent light emission is widely used for sensing and imaging of biological targets *in vivo* and *in vitro.* There are essentially three mechanisms for generating a light emission based optical signal: chemiluminescence, bioluminescence and fluorescence.

Chemiluminescence is the generation of electromagnetic radiation as light by the release of energy from a chemical reaction.

Bioluminescence is the production and emission of light by a living organism. Bioluminescence is exhibited by almost all main groups of organisms ranging in diversity from bacteria, fungi, and algae through to earthworms, squid and fish. All the light produced by these different organisms share a basic biochemical pathway. A chemical compound, known generically as luciferin, which is the light emitting substrate is modified by an enzyme, which is generally referred to as luciferase. Therefore, bioluminescence can be thought of as a chemiluminescence reaction that is catalyzed by an enzyme that occurs within a living organism.

Luciferases are a wide range of enzymes that catalyse the oxidation of substrate luciferins to yield non-reactive oxyluciferins and the release of photons of light. Luciferase is a generic name because none of the major luciferases share sequence homology with each other. There are five basic luciferin-luciferase systems: bacterial luciferin, dinoflagellate luciferin, vargulin, coelenterazine, and firefly luciferin. Five distinct chemical classes of luciferins are known to date, namely, aldehydes, benzothiazoles, imidazolopyrazines, tetrapyrroles and flavins. Coelenterazine, the most widely known luciferin, is an imidazolopyrazine derivative.

Fluorescence is the emission of light by a substance that has absorbed radiation of a different wavelength. In most cases, absorption of light of a certain wavelength induces the emission of light with a longer wavelength. The fluorescent molecule is generally called a fluorophore and no chemical reaction is involved in the emission of light following excitation.

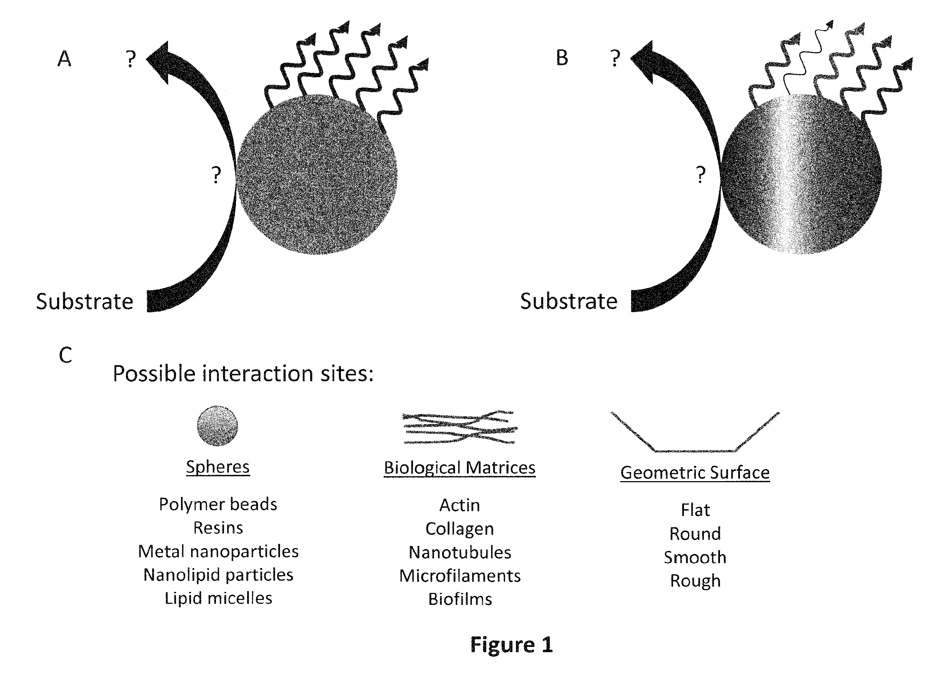

The inventors have shown that, unexpectedly, luminescent light is generated when bioluminescent substrates (luciferins) interact with a physical surface in the absence of any catalytic enzyme (luciferase). This phenomenon, named by the Inventors as Enzyme independent Photon Emission (EiPE), is illustrated on figure 1.

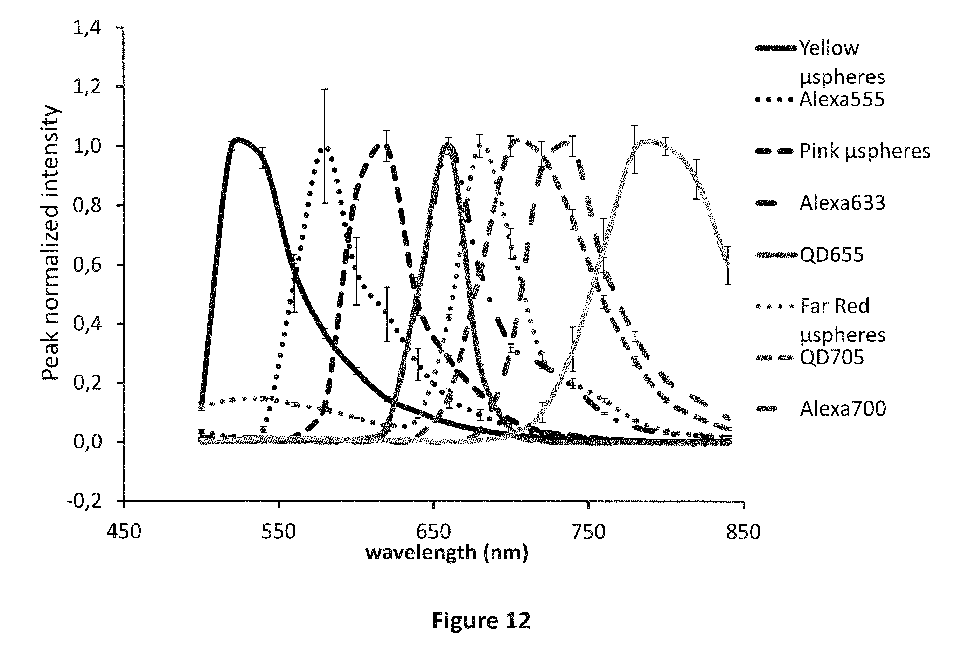

Furthermore, the inventors have shown that EiPE is able to stimulate light emission by a variety of organic and inorganic fluorophores covering the optical and near infrared spectrum, while preserving the spectral emission characteristics of the fluorophores exactly as if the molecules were excited in a conventional epi-fluorescent excitation process (figure 12).

Current results have demonstrated this phenomenon under *in vitro* conditions in both simple and complex media as well as *in vivo* conditions.

Without wishing to be bound by theory, current experimental evidence suggests that the process is due to a circuitous Resonance Energy Transfer (RET) between donor (substrate)-acceptor (fluorescent molecule such as fluorophore) molecules with high valence electron dense dipole states and interacting in a polar environment.

The use of EiPE to stimulate light emission by fluorescent molecule such as fluorophore offers several major advantages over conventional luminescent light emission systems:
- Constraints arising from fluorescent molecule such as fluorophore excitation spectra which are present in conventional epi-fluorescent excitation process do not exist in EiPE.
- As opposed to BRET/CRET, EiPE does not require optimized spectral overlap between donor (substrate) and acceptor (fluorescent molecule such as fluorophore).
- EiPE assures a Signal/Noise-ratio literally factor-fold higher than classical fluorescence because the photon-flux is low, requiring highly-sensitive low light level detectors (e.g. imaging systems or photon detection devices).
- EiPE signals do not photobleach at all, and EiPE signals are maintained stable during many hours, unlike fluorescence signals that suffer from excitation-illumination-dependent photo-bleaching that deteriorates peak light fluorescence signals over time, increasing noise and decreasing sensitivity.
- EiPE is compatible with spectral deconvolution methods and multiplexed assays in that it allows for a single substrate to trigger photon emission from multiple fluorescent molecules such as fluorophores whose distinct spectra are easily distinguished based upon the spectral characteristics of each fluorescent molecule such as fluorophore and can be detected using an appropriate combination of emission bandpass filters. This allows the detection of mixed signal components in the same sample.
- EiPE allows luminescent light emission from substrate to be harnessed and red-shifted to an extent determined by the specific emission characteristics of the chosen fluorescent molecule such as fluorophore. This process resembles closely the light output predicted from conventional epifluorescence where appropriate excitation light results in red-shifted emission light predictable from the *Stokes shift* characteristics of the chosen fluorescent molecule such as fluorophore. By contrast, classical chemiluminescence/bioluminescence is dependent upon a catalyst-driven consumption of substrate yielding luminescent light emission whose spectral characteristics are determined by the specificity of the substrate.
- EiPE does not need the genetic or chemical modification of the host, as opposed to bioluminescence.

EiPE is unique in that it offers a chemical alternative yet completely generic means to generate characteristic emission spectra from any given fluorescent molecule such as fluorophore.

This approach broadens the scope and application of low-light imaging and sensing of biological targets *in vitro* and *in vivo.*

Therefore, the invention concerns a method for generating a luminescent signal, comprising the step of contacting a bioluminescent substrate or reactive intermediate thereof with at least one physical surface in the absence of a catalytic enzyme, wherein the interaction of said bioluminescent substrate with said physical surface(s) generates a detectable luminescent signal.

According to the present invention, the catalytic enzyme refers to the enzyme which catalyses the oxidation of said bioluminescent substrate to yield non-reactive oxydated substrate and the release of photons of light.

According to the present invention, the reactive intermediate refers to a compound formed as an intermediate during the reaction of a bioluminescent substrate with a catalytic enzyme.

In the present invention, the bioluminescent substrate is referred to as luciferin and the catalytic enzyme as luciferase.

According to the present invention, the physical surface refers to any matter able to present a physical interface able to adsorb or covalently interact with any organic or biological molecule.

According to the present invention, the luminescent signal represents all the light that is generated by the interaction of the bioluminescent substrate with the physical surface. The "basal signal" results from the interaction of the substrate with the surface (Figure 1A); it depends on the luminescence emission spectrum of the substrate. For example, decanal, coelenterazine and its derivatives (h, hcp and fcp) have a natural blue emission. If the physical surface comprises a fluorescent molecule, a "wavelength-specific signal" is emitted (Figure 1B). The "wavelength-specific signal" is characteristic of the emission spectrum of the fluorescent molecule. Without wishing to be bound by theory, it is believed that the luminescent signal (basal signal) generated by the reaction of the bioluminescent substrate with the physical surface excites said fluorescent molecule, which emits a detectable luminescent signal (wavelength-specific signal).

Unless otherwise stated, "substrate" refers to "bioluminescent substrate" and "surface" refers to "physical surface".

The method of the invention may be performed *in vivo* in a living organism (animal or plant), or *in vitro* (outside a living organism). In *vitro* means in a living cell, or in a test tube, or any other artificial environment. The cell may be any cell including a unicellular organism (bacteria, yeast,..), a cell from a cell culture, or a cell that has been taken from a living organism.

The invention may be performed using any luciferin from any classes of luciferins, *i.e.,* aldehydes, benzothiazoles, imidazolopyrazines, tetrapyrroles and flavins.

According to a preferred embodiment of the method of the present invention, the luciferin is chosen from aldehydes and imidazolopyrazines, preferably from decanal (decanaldehyde), coelenterazine (6-(4-hydroxyphenyl)-2-[(4-hydroxyphenyl)methyl]-8-(phenylmethyl)-7H-imidazo[3,2-a]pyrazin-3-one; Substituents groups R1, R2 and R3, in positions 2, 6 and 8 are hydroxyl (OH), hydroxyl (OH) and Phenyl (Phe), respectively) and its analog derivatives. Non-limitative examples of coelenterazine analogs suitable for the method of the invention include coelenterazine h (2-deoxy derivative of native coelenterazine; R1=H, R2=OH and R3=Phe), coelenterazine hcp (R1=H, R2=OH and R3=CP (Cyclopentyl)) and coelenterazine fcp (R1=F (Fluorine), R2=OH and R3=CP (Cyclopentyl)).

The physical surface may be the surface to which a fluorophore molecule binds (*i.e*. different from the substrate) and might be any surface of any material used in biological assays. For example, the physical surface may be that of a fluorescent molecule including a fluorophore, or a fluorophore conjugated to a molecule such as a protein. Materials suitable for the method of the invention include with no limitation: polymer beads, resins, metal nanoparticles, nanolipid particles, lipid micelles and biological matrices. Non-limitative examples of biological matrices include actin matrices, collagen matrices, microtubules, microfilaments, and biofilms. In some embodiments, the material has a high surface/volume ratio. The surface may be flat, round, smooth or rough. In addition, the surface of the material may be modified, for example functionalized with a chemical group and/or compound including for example a molecular probe and/or a fluorescent molecule, or magnetized; surface modification(s) may be used to improve the luminescent signal.

According to another preferred embodiment of the method of the present invention, said physical surface is the surface of a particle. Preferably, a metal, lipid, resin, and/or polymer microsphere or nanoparticle, such as a polystyrene microsphere, lipid nanoparticle, or lipid micelle. In addition, the particle may be magnetized.

According to another preferred embodiment of the method of the present invention, said physical surface is the surface of a biological matrix, preferably chosen from an actin matrix, a collagen matrix, a microtubule, a microfilament or a biofilm.

According to another preferred embodiment of the method of the present invention, said physical surface comprises a fluorescent molecule and said detectable luminescent signal is that emitted by said fluorescent molecule (wavelength-specific signal). As explained just above, it is believed that the luminescent signal (basal signal) generated by the interaction of the bioluminescent substrate with the physical surface excites said fluorescent molecule, which emits a detectable luminescent signal (wavelength-specific signal). Light emission maintains the spectral emission characteristics of the fluorescent molecule. In some embodiments, the fluorescent molecule is bound to the physical surface by appropriate means, which are known in the art. When the physical surface is that of a particle, the fluorescent molecule may be bound to the surface and/or incorporated inside the particle. The fluorescent particle is advantageously a quantum dot, a fluorescent polystyrene microsphere, or a fluorescent lipid nanoparticle. The fluorescent molecule is preferably a fluorophore. The physical surface is advantageously coated with a molecular probe specific for a target of interest (capture-probe). The method may further use a target-specific detector-probe, either labeled with a fluorophore, or biotinylated or used in combination with a streptavidin-conjugated fluorescent molecule, for example.

Preferably, the method is performed with several fluorescent molecules of different emission wavelength, preferably fluorescent molecules covering the optical and near infrared spectrum, wherein each fluorescent molecule is bound to a separate physical surface, advantageously a separate particle. This allows the emission of different luminescent signals by the different fluorescent molecules, preferably fluorophores, which can be used to detect different signals. Multiplexed assays allow the detection of mixed signal components in the same sample. They provide a highly sensitive detection of targets *in vitro* or *in situ* under a great variety of conditions.

The method of the invention may be used for the detection of any target of interest using standard assays, which are known in the art. These assays are based on the detection of the target *in vivo* in a living organism (animal or plant) or *in vitro,* using a molecular probe that binds to the target. The molecular probe may be freely diffusing, or attached to beads in flow, or attached to diffusion columns, or attached to slide/well/micro-pattern substrates. *In vitro* assays are performed in various formats such as wells, slides, microspots, and beads. Examples of these assays are enzyme assays, nucleic acid assays, receptor-ligand assays and immunoassays. Immunoassays are widely used assays based on the detection of the target with specific antibodies.

The method of the invention is advantageously used for the detection of biomarkers useful in research, biotechnology, diagnostics, therapeutics, genetic analysis and/or drug-screening.

The luminescent signal is detected using appropriate systems, preferably highly-sensitive low light level detectors which are well-known in the art (photomultiplier, photocathode, CCD camera, enhanced CMOS/CDD camera).

Another aspect of the present invention concerns a kit for performing the method according to the present invention, comprising:
- at least one bioluminescent substrate according to the present invention,
- at least one physical surface according to the present invention, preferably comprising a fluorescent molecule, more preferably coated with a molecular probe specific for a target to detect, even more preferably a particle, and
- instructions for the performance of the method according to the present invention, and wherein the kit does not comprise any catalytic enzyme (luciferase).

Another aspect of the invention relates to the use of a bioluminescent substrate, in the presence of at least one physical surface according to the present invention, and in the absence of a catalytic enzyme, to produce a detectable luminescent signal. Preferably, said bioluminescent substrate is used with a physical surface comprising a fluorescent molecule to stimulate the emission of a detectable luminescent light signal by the fluorescent molecule(s).

In some embodiments, the method and use of the present invention for diagnostics and/or therapeutics purposes are performed *in vitro;* the method and use of the present invention for other purposes are performed *in vitro* or *in vivo.*

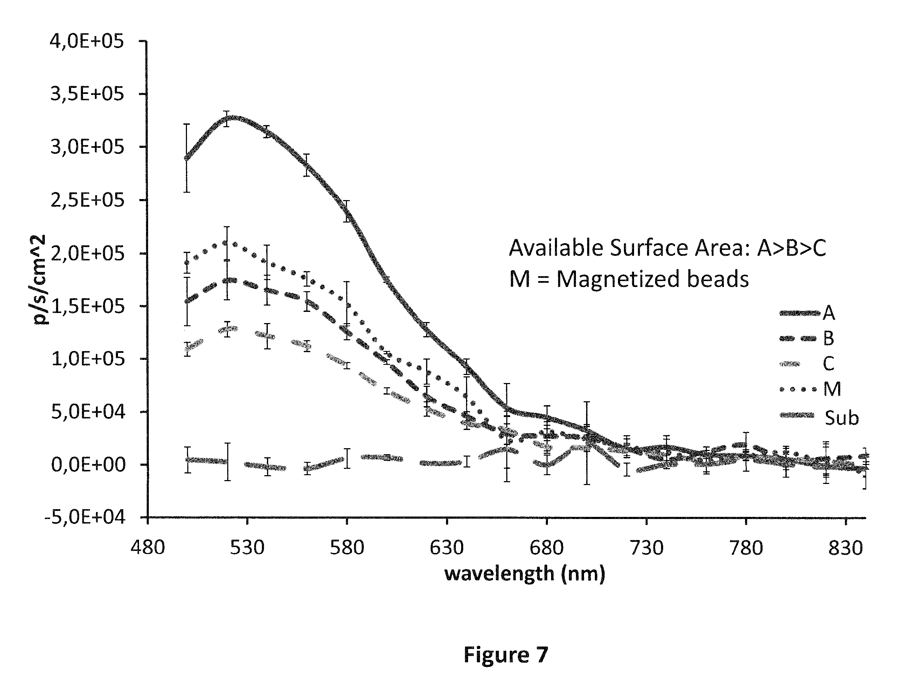

The potential applications of EiPE are broad, based on the ability of this method to facilitate high-sensitivity multi-valent detection of specific signals using existing and new probes. In particular, EiPE can help enhance and optimise existing methods for affinity separation (cells and other targets).
- Visualization of targeted biomolecules *in vitro* and *in situ:*
   "Enzyme independent" EiPE by definition might serve to replace the enzyme-linked antibody detection used in classical ELISA. The advantages of EiPE compared with an enzyme-linked antibody are anticipated to be greatly enhanced sensitivity, and
   multiple signal detection. Enzyme linked assays are able to amplify just one, or two specific signals so that they become detectable with enhanced contrast. EiPE detection using fluorescent labels opens the way for multiple signals to be simultaneously detected using spectral detection methods, and integration of the photonic signal over time.
- Cell and organelle separation techniques : Isolation of cells, organelles, and proteins from complex mixtures such as blood is important to basic research, biotechnology, diagnostics, therapeutics, genetic analysis, and other applications. Toward these ends *flow-fractionation* methods are pivotal; magnetic and dielectric approaches can be combined with molecular recognition (for example, prelabeling of cells with magnetic or dielectric micro-particles) in order to separate cells. As shown in Figure 7 (label "M") under the appropriate conditions robust EiPE light is generated from magnetized microscopic beads. Such microscopic beads can easily be functionalized by covalent attachment of antibodies targeting specific cell subpopulations based on surface markers, and used for magnetic cell separation. Equally, all types of antibody attached magnetic particles themselves are fully expected to produce EiPE because of their large diameter (0.1 - 10 micron). In all these cases EiPE could serve a specific means for semi-quantitative real-time monitoring of, for example, magnetic particles measuring directly the accumulation of magnetic-particlebound cells inside the magnetic field using sensitive optical detection. For studies using blood samples it is evident that magnetic particles should better be doped with far-red fluorophore and the EiPE detection thereby optimized inasmuch as blood is transparent to red photon light of spectral wavelength superior to 700nm, but not bluegreen light in the range inferior to 650nm.

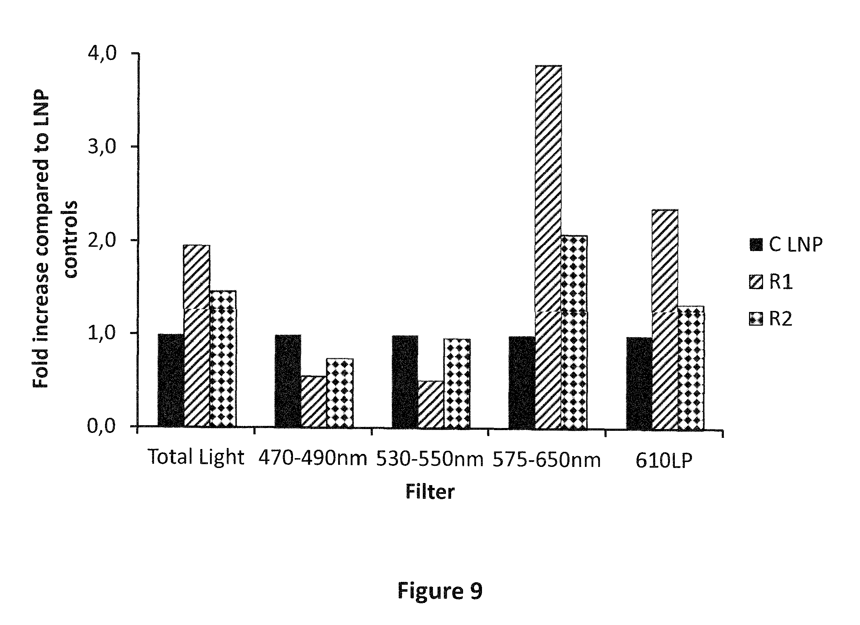

In addition to, and compatible with magnetic/di-electric particle based cell separation techniques cell affinity chromatography, type-specific cell separation is based on the interaction between cell-surface receptors and an immobilized ligand on a stationary matrix. For example, assembled monolithic polyacrylamide and polydimethylacrylamide cryogel affinity matrices can be used to construct monolithic cell affinity columns comprising highly interconnected pores (up to 100 µm) for convective migration of large particles such as mammalian cells. Such columns may be functionalized to immobilize cells using, for example, *Protein-A.* Target cells labelled with specific antibodies are captured in the affinity column these specifically captured cells are recovered at high yields, and viability by elution. Cell separation using affinity chromatography is also enhanced by the implementation of EiPE in a diversity of ways. For example, EiPE compatible antibody labelled particles susceptible to producing EiPE can reveal directly by optical means the specific accumulation of labelled cell subpopulation in the affinity column. For example, affinity column targeting could be multiplexed with other biomarker readouts using EiPE producing micro-particles of spectrally distinct (smaller) size to detect a second biomarker, for example, a disease marker.
- *In vitro, in situ,* and *in vivo* analysis of nanoparticle targeting theranostic application: An emergent field in biological chemistry concerns nanoparticle delivery, which promises to provide high specificity targeted payload delivery *in vivo.* This type of approach has enormous potential to increase the armoury of small molecules that could be used for cancer, and infection. The EiPE method holds promise to facilitate such emerging technologies in a number of different Indeed, the figure examples show that EiPE signals are implicit to a variety of chemically and physically diverse types of nanoparticle including: lipid nanoparticles, polystyrene microsphere particles, synthetic resins and inorganic quantum dots. It is therefore clear that EiPE will provide an important tool for screening, developing, validating, and ultimately applying nanoparticle technology for nanomedicine and theranostic applications. The possibilities can be illustrated considering the facile yet powerful ability to tune the spectral characteristics of EiPE signal such that nanoparticles may be given specific unique spectral fingerprints depending upon limitless possibilities where endless combinations of fluorphore could allow unique spectral "barcodes". While *EiPE spectral properties* are an independently tuneable parameter, *EiPE intensity* is dependent upon physiochemical properties of the particles including, for example, surface/volume, interlinking, and perhaps most importantly functionalization state of the outermost solid-phase interface. For example, Table II, shows the same types of polystyrene bead produce different EiPE signal intensity depending upon surface functionalization (streptavidin, amine). Such EiPE-based tools could be useful in screening *in vitro* and *in vivo,* and even theranostic applications. For example, *lipid nanoparticles* (LNPs) are an therapeutic delivery technology. Figure 9 shows that all LNP yield abundant EIPE. In this context, LNP-EiPE will be of considerable value for the *in vitro* development, validation and eventual *in vivo* detection of LNP. It will be highly *advantageous* to be able to monitor the distribution and targeting of custom LNPs using optical imaging methods, especially to define their efficacy - EiPE would facilitate this type of analyses.
- *In situ* analysis of biomolecule distribution

Nanomedicine draws upon diverse expertise including: theranostic medicine, chemical biology, biophysics, immunology, nanoparticle production, drug screening and cell biology/physiology. Therein monitoring biomolecule distribution in *situ* using small animal models is an inherent part of the validation process approved by health authorities. The current state-of-the-art for pre-clinical studies to establish, for example, drug (small molecule) distribution uses Radio-immunoassay (RIA) in small animal models (for example, mice). Animals are first exposed to a radiolabelled small molecule (for example, by *i.v*.), and after a period of time euthanized, frozen whole, and then prepared by cryomacrotome to produce whole animal tissue thin-sections. The whole animal can be cut into hundreds of thin sections (between ten and several hundred microns thick), which are then individually imaged using a dedicated radio-imaging device capable to quantify the radiolabel signal and extrapolate the accumulated concentration of the target molecule under study. Inasmuch as production of radionucleotides is expensive, time-consuming, and pose a laboratory risk factor it would be advantageous to replace radiolabeling with photon-detection based methods. EiPE could today be conceivably used in this context for small molecule tracking. However, combined with the method of whole animal thin slice analyses EiPE holds a perhaps much greater promise.

For a better understanding of the invention and to show how the same may be carried into effect, there will now be shown by way of example only, specific embodiments, methods and processes according to the present invention with reference to the accompanying drawings in which:
- **Figure 1****. Schematic depicting the EiPE phenomenon.** Upon the interaction of the substrate and an appropriate surface, a basal signal is generated (A). If a fluorophore is present, a wavelength-specific signal is observed (B). Non-limitative examples of interaction surfaces appropriate for EiPE (C).

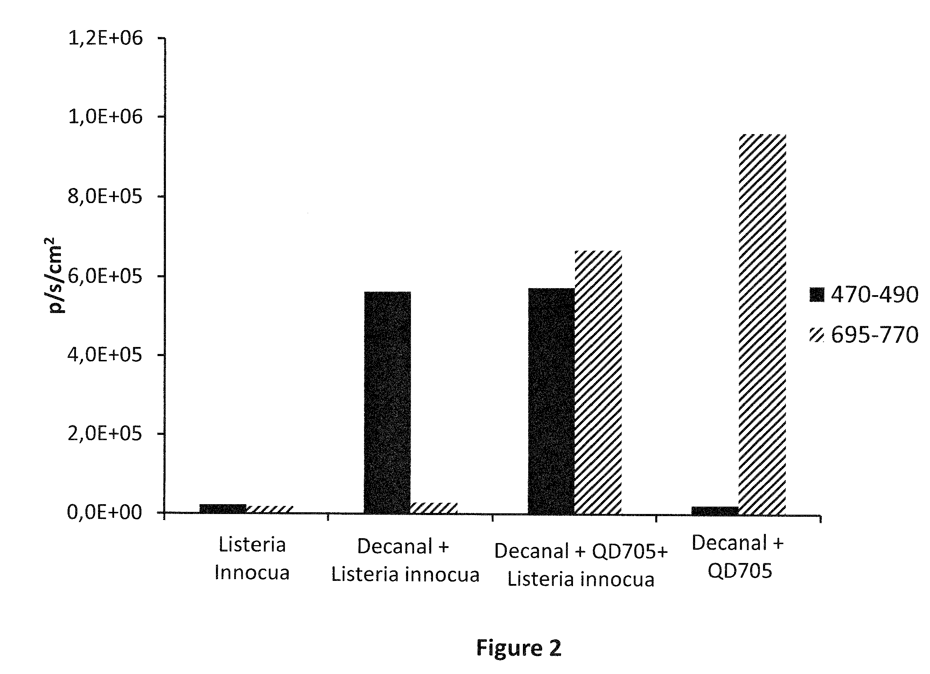

- **Figure 2****. Initial Evidence of the Existence of EiPE.** Using combinations of *Listeria* inoculate, decanal, and QD705 in PBS various solutions were formed as indicated and then dispensed into a black 96-well plate. The plate was then placed into an IVIS100 bioluminescence imaging system and observed under the 470nm - 490nm, and 695nm - 770nm filter sets. Luminescence was observed for the combinations of decanal + *Listeria* innocua, decanal + *Listeria* innocua + QD705, and for decanal + QD705. Blue-specific signal was found only when the *Listeria* inoculate were present. Further, QD705-specific signal was seen whenever the QD705 were mixed with the decanal, even in the absence of the *Listeria* inoculate. It is this final observation that led to the discovery of Enzyme-independent PhotonEmission (EiPE).

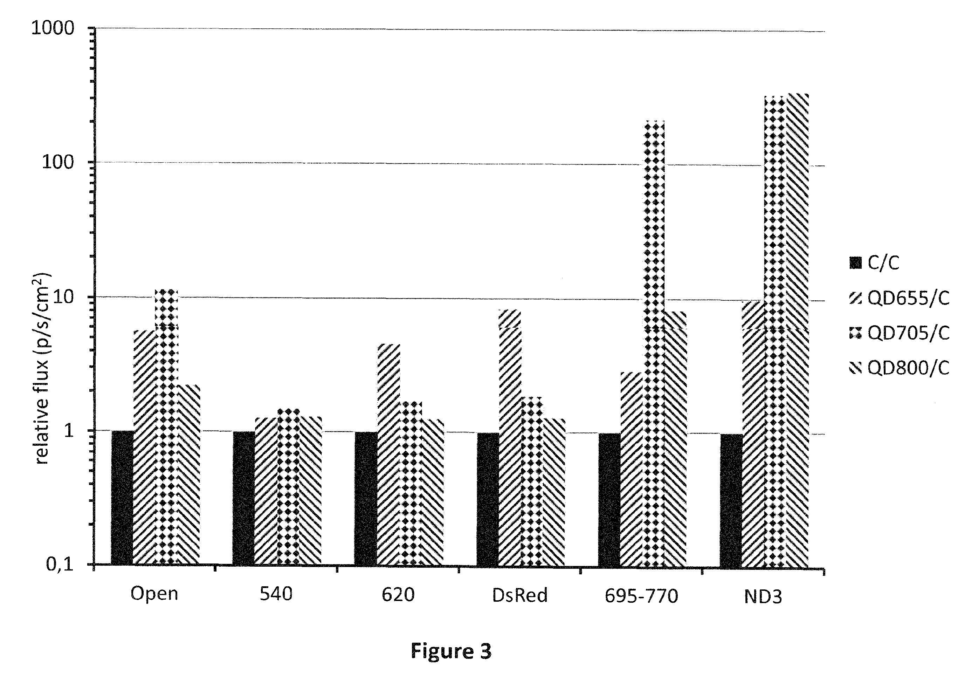

- **Figure 3****. Wavelength-Specific Signal Generation Using EiPE with Various QDs.** Solutions containing only the substrate, in this case Coelenterazine-h, or substrate and QD655, QD705 or QD800, were prepared in 1x PBS and dispensed into *Eppendorf* tubes. The tubes were then placed into an IVIS 100 bioluminescence imaging system and observed under the Total Light, 530-550nm, 610-630nm, 575-650nm, 695-770nm, and 810-870nm emission filters. Wavelength-specific signal was observed for each solution that contained the QDs, with the maximum emission observed under the correctly corresponding emission filter. The signal of each solution was compared to the substrate control, and a massive increase in signal was observed. This increase approached nearly 300-fold when the QD705 and QD800 were used, indicating the massive specificity of the signal.

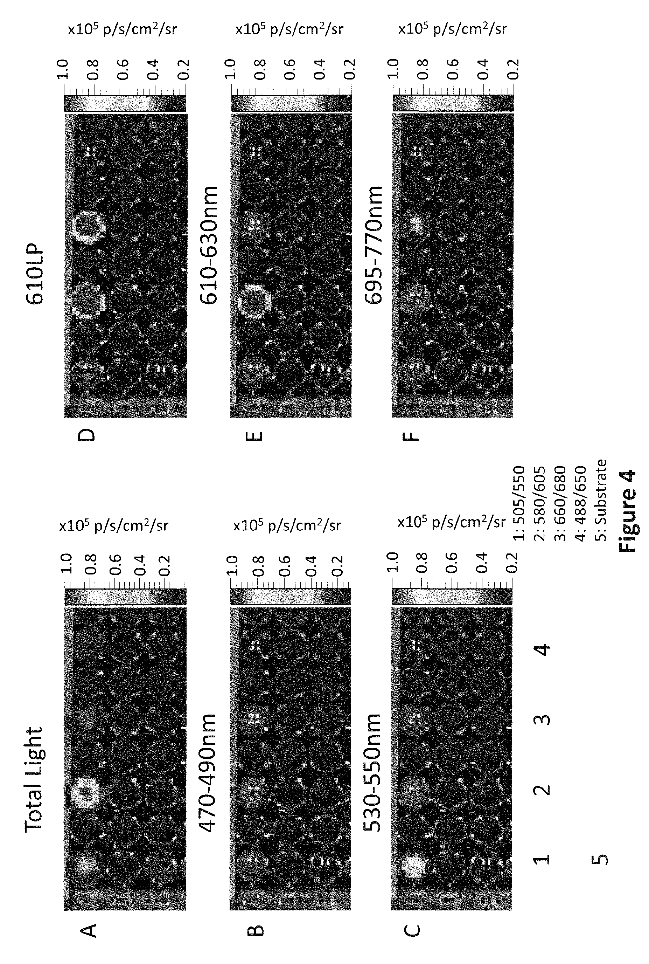

**Figure 4****. Wavelength-Specific Signal Generation Using EiPE with Various Fluorophore-Embedded Polystyrene Microspheres.** Solutions containing only the substrate, in this case Coelenterazine-h, or substrate and polystyrene microspheres embedded with various fluorophores, were mixed with 1x PBS and dispensed into individual wells of a black 96-well plate. The fluorophore-embedded microspheres had the following excitation/emission maxima: 505nm/550nm, 580nm/605nm, 660nm/680nm, and 488/650nm. The plate was then placed into an IVIS100 bioluminescence imaging system and observed under the Total Light, 470-490nm, 530-550nm, 610 Long Pass, 610-630nm and 695-770nm emission filters (A-F, respectively). Wavelength-specific signal was observed for each solution that contained the polystyrene microspheres, with the maximum emission observed under the correctly corresponding emission filter.

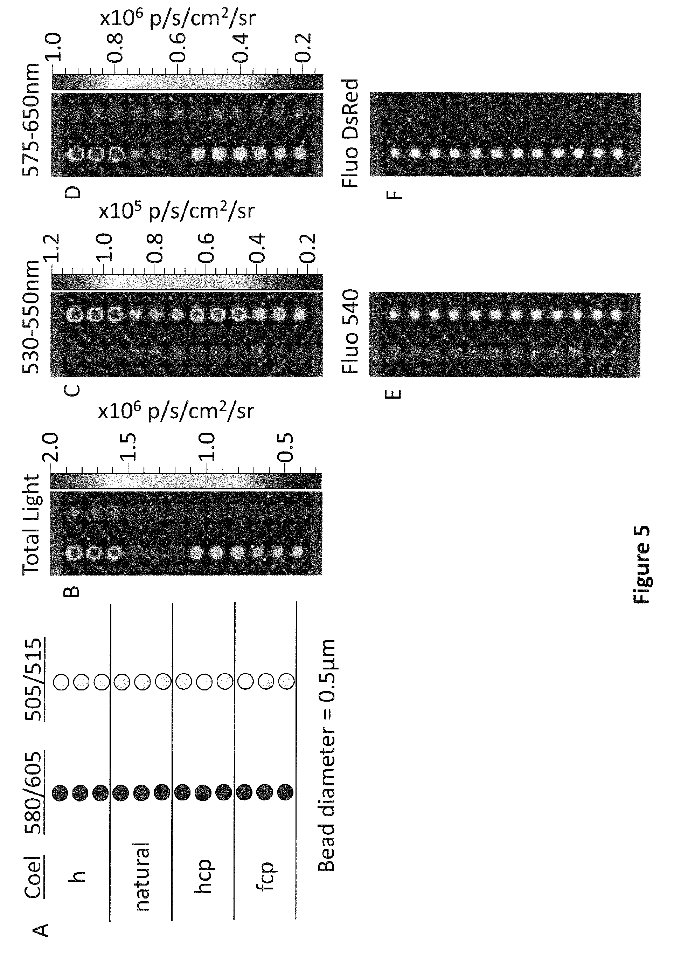

- **Figure 5****. The Effect of Different Substrates on Light Generation Using EiPE.** The effect of different substrates on the ability to generate light using EiPE was investigated by mixing Coelenterazine or one of its derivatives with fluorophore-embedded microspheres. The light generated from two different fluorophore-embedded microspheres mixed with the commonly used coelenterazine derivatives h, hcp, and fcp were compared to the native form. The solutions were prepared in triplicate and dispensed into a black 96-well plate (A). The plate was placed into an IVIS100 bioluminescence imaging system and observed under the Total Light, 530-550nm, and 575-650nm emission filters (B-D, respectively). The microsphere location was confirmed using epifluorescence (E and F). As anticipated, the wavelength-specific signal is generated and confirmed to appear under the appropriate emission filters. Interestingly, the amount of signal generated varied from each derivative and for each fluorophore used. This indicates that for a given fluorophore used there exists an ideal derivative that promotes maximum signal generation.

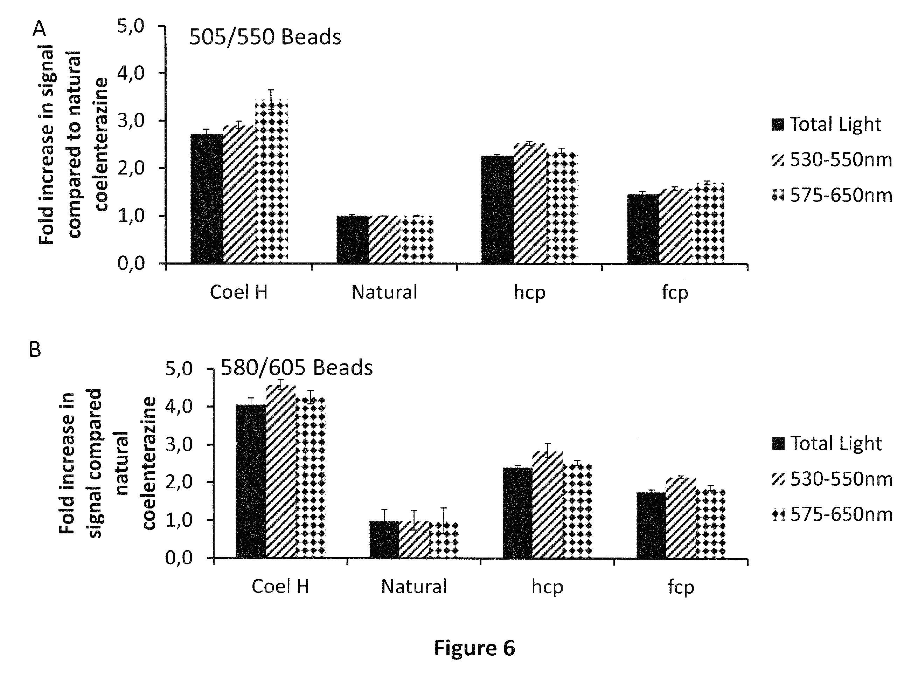

- **Figure 6****. Analysis of EiPE Substrate Dependence (****Figure 5****).** The resulting photons/s/cm² from each acquisition as shown in Figure 5 was normalized to the respective coelenterazine control, labeled as natural for each condition. Similar trends were observed for the 505/550 (A) and 580/605 (B) carboxylate functionalized fluorophore-embedded polystyrene beads. The greatest signal enhancement was observed when coelenterazine-h was used, and each derivative generated at least twice as much signal as the control. This result implies that further chemical modifications to the coelenterazine backbone may yield an increased in generated luminescence.
- **Figure 7****. Changing the Surface Area of ReSyn® Beads and its Effect on EiPE Signal Generation.** A ReSyn® bead consists of a long polymeric chain that freely and naturally winds around itself generating a microsphere with a massive surface area to volume ratio. As such, the polymer generates a bead-like structure that is extremely porous and allows the free movement of liquids throughout its entirety. ReSyn® beads were fabricated such that they had varying degrees of crosslinking, thus regulating the amount of surface area available for the EiPE reaction. In triplicate, solutions containing equal concentrations of the respective ReSyn® microspheres and the coelenterazine-h substrate in 1x PBS were prepared and dispensed into a black 96-well plate. The resulting luminescence was observed using an IVIS Spectrum under all the available filter sets (20nm band pass filters which encapsulate the emission from 490nm to 850nm). The emission under the 490-510nm filter is shown (inset). As is indicated, ReSyn® A had the the largest amount of available surface area, followed by ReSyn® B and then ReSyn® C. ReSyn® M is a magnetic variant of ReSyn® A, whereas Subindicates a solution where no beads are present but only the substrate in 1x PBS. While the resulting emission maxima for each ReSyn® bead occurred at the same region (510-530nm), the intensity of the emission is strongly correlated to the degree of crosslinking.

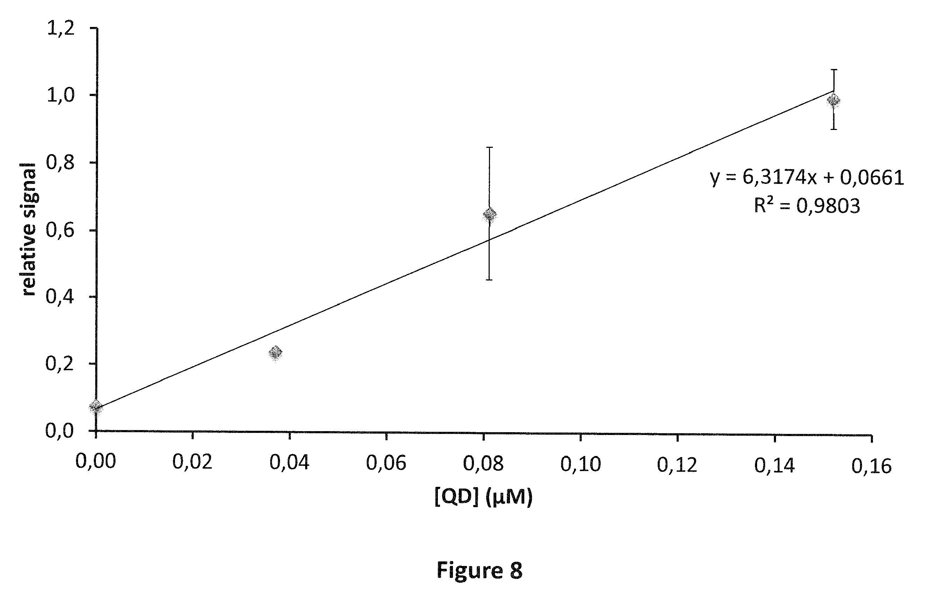

- **Figure 8****. Wavelength-Specific Signal Generation Using EiPE with QD705-Labelled ReSyn® Beads.** In triplicate, equal concentrations of ReSyn® Beads labeled with various concentrations of QD705 and mixed with the coelenterazine-h substrate in 1x PBS were distributed into a black 96-well plate and observed under the 710-730nm filter set of an IVIS Spectrum (Top). The resulting p/s/cm² were normalized to the average value of the beads that produced the most signal (0.152µM QD705, Bottom). As can be seen from the plotted data, the most intense QD705 signal occurred in the case where the concentration was at the highest. The resulting luminescence decreased in a linear manner.
- **Figure 9****. Wavelength Specific Signal Generation Using EiPE with Rhodamine6-Loaded Lipid Nanoparticles (LNPs) from Global Acorn®.** Three formations of lipid nanoparticles from Global Acorn were mixed with coelenterazine-h in 1x PBS and distributed into a black 96-well plate. The three formations consisted of a control (ie, no fluorophore attached nor on the interior) LNP (C LNP), with rhodamine-6 both bound to the surface and loaded into the center of the LNP (R1), and with rhodamine-6 loaded into the center only (R2). The plate was placed into an IVIS100 bioluminescence imaging system and observed under the Total Light, 470-490nm, 530-550nm, 575-650nm, and 610 long pass (LP) emission filters (Top images). The relative p/s/cm² for each LNP under each filter set was normalized to the control LNP (Bottom). As can be seen, the LNP that was both labeled with and contained the rhodamine-6 produced the greatest amount of signal under the Total Light, 575-650nm, and 610LP emission filters. This demonstrates that there exists the possibility to create biocompatible polymeric backbones to which a fluorophore or a fluorescent nanoparticle may be attached, and the wavelength of the luminescence predicted.

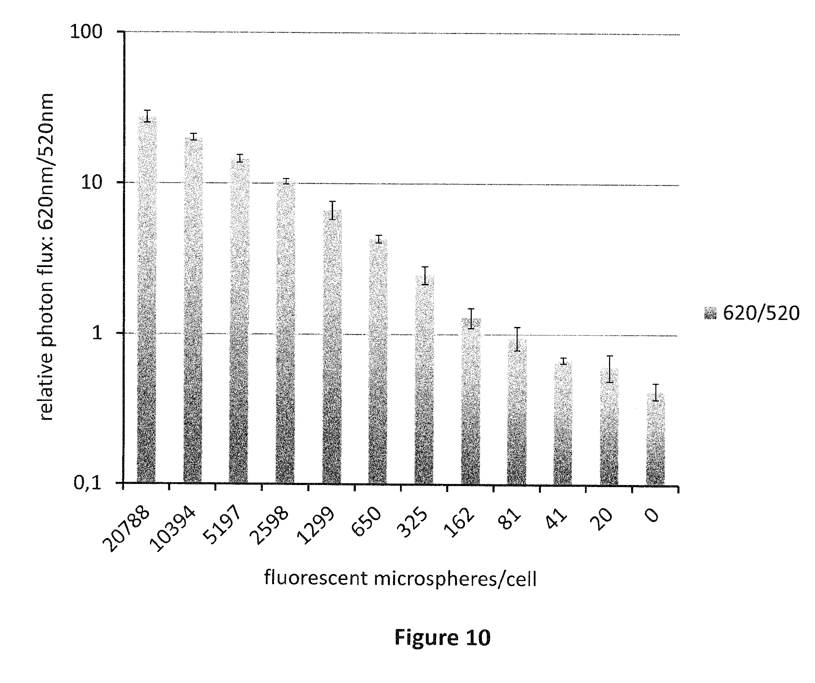

- **Figure 10****. Signal Generation Using EiPE with Fluorophore-Embedded Microspheres in the Presence of Fixed J774A.1 Murine Macrophages in Suspension.** An equal number of fixed J774A.1 murine macrophages were distributed into the wells of a black 96-well plate. Into the same wells, a decreasing number of 0.5 µm fluorophore-embedded microspheres with maximum excitation and emission wavelengths of 580 and 605 nm, respectively, were distributed such that the microsphere-to-macrophage ratio ranged from 20788 to 20, with one well kept free from microspheres (noted as 0). After the microspheres were distributed, equal volumes of coelenterazine-h in 1x PBS were added to each well and the plate placed immediately into an IVIS Spectrum bioluminescence imaging system. The plate was observed under all available emission filters (ranging from 490nm - 850nm). The total photons/second/cm² were determined for each well under each filter. The resulting total flux at 610 - 630 nm for each well was normalized by the flux found at 510-530 nm, and the resulting enhancement in red signal displayed. Each condition was repeated in triplicate. As can be seen, a substantial wavelength-specific signal was generated whenever the beads were present, with the magnitude of the signal dependent upon the microsphere-to-macrophage ratio. The microsphere-specific signal is clearly discernable from the background at as few as 20 microspheres per macrophage.

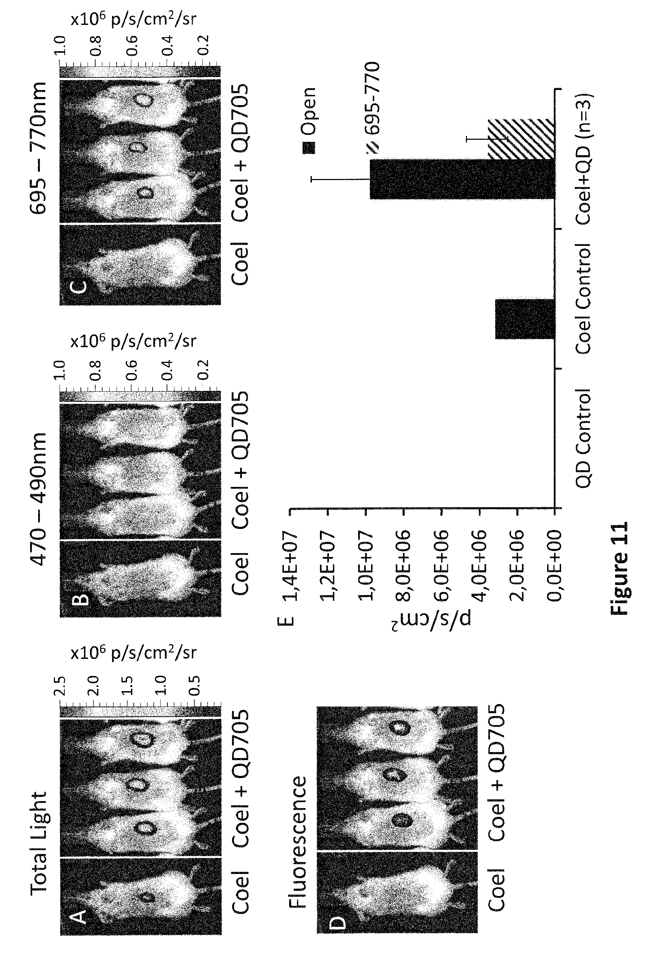

- **Figure 11****. The Existence of EiPE under *in vivo* Conditions.** The dorsal side of four 6-week old female BALB/c mice was razed and the mice placed under general anesthesia. One solution containing only coelenterazine-h in 1x PBS was prepared and used as the control (Coel). A second solution containing the coelenterazine-h in 1x PBS along with QD705 was prepared (Coel + QD705). One mouse received one subcutaneous injection of the Coel control and was imaged under the Total Light, 470-490nm, and 695-770nm emission filters. The three other mice received identical volume injections of the Coel + QD705 solution and were also imaged under the same filter sets (A-C). The QD705 location was confirmed by epifluorescence (D). The resulting p/s/cm² were compared under each filter set (E). As can be seen, nearly a three-fold increase in total signal is observed when the Coel + QD705 solution is present compared to the Coel control. This contrast is even stronger when the QD705-specific filter is used, providing a nearly 20-fold increase in signal. **-** **Figure 12****. Using EiPE to Generate Fluorophore-Specific Signals from Multiple Luminescent Sources Covering Nearly the Entire Optical Spectrum.** Coelenterazine-h in 1x PBS was mixed with a multitude of fluorophores and fluorescent nanoparticles, including 505/550nm, 580/605nm, and 660/680nm polystyrene microspheres (Yellow, Pink, and Far Red, respectively); QD655, QD705, and QD800; as well as unbound streptavidin-functionalized Alexa dyes (555, 633, and 700). The solutions were produced in triplicate and distributed into a black 96-well plate. The plate was observed in an IVIS Spectrum bioluminescence imaging system under all available filter sets (ranging from 490nm - 850nm). The entire emission spectrum for each fluorophore was acquired and normalized to the peak emission wavelength. As can be seen, the appropriate fluorescence emission spectrum of each compound was achieved using EiPE.

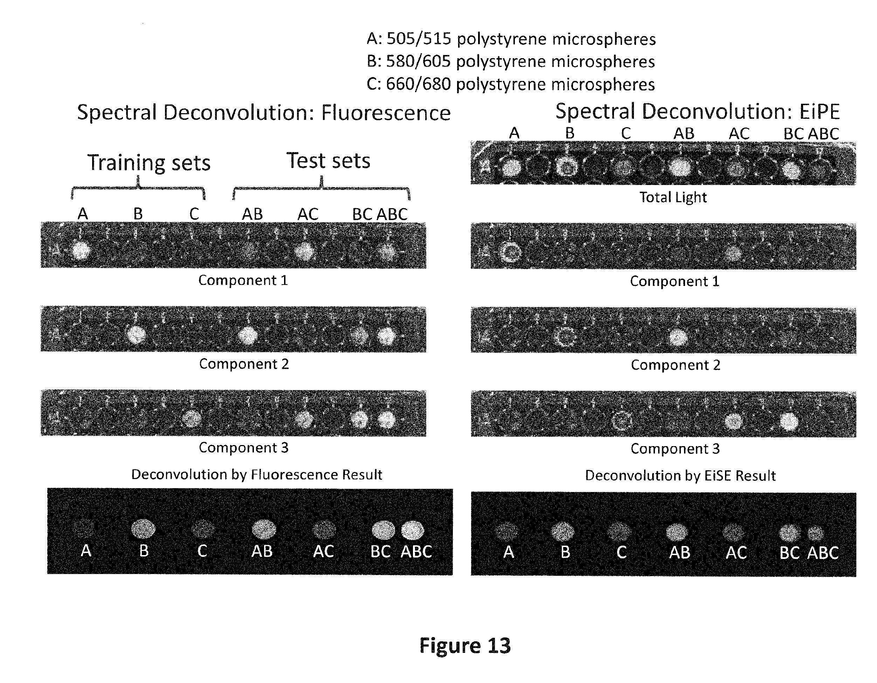

- **Figure 13****. Multiplexed spectral deconvolution using EiPE.** Polystyrene beads doped with one of three fluorophores (A,B,C) were mixed v/v (AB,AC,BC,ABC) in a 96 well plate in the presence of Coelenterazine-h and treated to reveal EiPE light. Multi-well plate was visualized first using fluorescence illumination (*left panels)* as a control, and next using EiPE light generation *(right panels*)*.* Spectral deconvolution was performed using 8 filter sets covering the visual spectrum range of 490 to 730 nm with 20nm bandwidths. The three "component" channels Spectral deconvolution revealed the presence of the beads both alone and mixed together identically for fluorescence and EiPE illumination.

### Example 1: Materials

### Common reagents

Coelenterazine-h was acquired from two different commercial sources (SigmaAldrich, France, and Zymera, Inc, USA). Coelenterazine (natural) and other coelenterazine derivatives (hcp, and fcp) were acquired from SigmaAldrich (France). Unless otherwise stated, the coelenterazine-h used was provided by Zymera, Inc. All the coelenterazines were immediately dissolved in 1,2-propanediol (SigmaAldrich, France) upon reception to a concentration of 0.5 mg/mL. The resulting solution was dispensed into 50µL aliquots and kept at -20 °C until use. Prior to use, the coelenterazines were diluted 10-fold to a final stock concentration 0.05 mg/mL.

The decanal (SigmaAldrich, France) was stored in the dark until use.

The quantum dots (2 mM solutions) and the fluorescent polystyrene microspheres (2% solids) were acquired from Life Technologies (USA) and used as received. Custom polymeric microspheres were provided by ReSyn®, LLC (South Africa), and custom lipid nanoparticles were provided by Global Acorn (United Kingdom).

**Table I: Summary of the commercial nanoparticles used within the respective figures**

| **Name of Material/ Equipment** | **Company** | **Catalog Number** | **Comments/ Description** | **Concentration** |
|---|---|---|---|---|
| Q-Tracker 655 | Life Sciences | Q21021MP | | 2 µM |
| Q-Tracker 705 | Life Sciences | Q21061MP | | 2 µM |
| Q-Tracker 800 | Life Sciences | Q21071MP | | 2 µM |
| Alexa 555 | Life Sciences | S21381 | | 1 mg/mL |
| Alexa 568 | Life Sciences | S11226 | | 1 mg/mL |
| Alexa 633 | Life Sciences | S21375 | | 1 mg/mL |
| Alexa 700 | Life Sciences | S21383 | | 1 mg/mL |
| Pink microspheres | Life Sciences | F8887 | 40nm diameter | 1 % solids |
| Yellow microspheres | Life Sciences | F8888 | 40nm diameter | 1 % solids |
| Far Red microspheres | Life Sciences | F8789 | 40nm diameter | 1 % solids |

### Bioluminescence imaging

An IVIS100 or an IVIS Spectrum, both provided by Perkin Elmer, was used to acquire all the luminescence images. The IVIS100 was equipped with multiple band pass filters as indicated in the figures. Total Light indicates the absence of an emission filter, thus allowing for all the emitted wavelengths to be detected. The IVIS Spectrum contains 18 band pass filters with each having a nominal bandwidth of 20 nm, ranging from 490 nm - 850 nm. Each system provided the capability of standard epifluorescence imaging. The acquired data was analyzed using the provided software, LivingImage, versions 4.1 and 4.2.

### Macrophage

J774A.1 murine macrophages (ATCC) were grown under standard conditions (DMEM with 10% fetal bovine serum).

### Modified bacteriophages

Modified bacteriophages that contained the genetic material to induce luxAB expression from infected *Listeria* were prepared using standard molecular biology techniques.

### Mice

6-week old female BALB/c mice were acquired from Janvier (France) and kept under standard growing conditions following all European Union rules and regulations concerning animal ethics. Immediately after experimentation, the mice were sacrificed and appropriately disposed of.

### Example 2: Initial Evidence of the Existence of EiPE

### 1. Methods

Aliquots from a fresh overnight culture of *Listeria* were exposed to modified bacteriophages that contained the genetic material to induce luxAB expression, for up to 2 hours. After exposure, three 100 µL aliquots of the *Listeria* and bacteriophage mixture were dispensed into individual wells of a black 96 well plate. Of these wells, 10 µL of decanal was added to one well while a second received 10 µL of decanal and 10 µL of QD705. As a control, nothing was added to the third well. A solution containing 100 µL of 1x PBS, 10 µL of decanal, and 10 µL of QD705 was added to a fourth well. The prepared plate was placed into an IVIS100 and observed under the Total Light, 470 nm - 490 nm, and 695 nm - 770 nm filter sets using an exposure time of 30 seconds. The location of the QD705 was verified using epifluorescence.

### Results

Using combinations of *Listeria* innocua, decanal, and QD705 in PBS (Figure 2) various solutions were formed and then dispensed into a black 96-well plate. The plate was then placed into an IVIS100 bioluminescence imaging system and observed under the Total Light, 470nm - 490nm (figure 2), and 695nm - 770nm filter sets (figure 2). Luminescence was observed for the combinations of decanal + *Listeria* innocua, decanal + *Listeria* innocua + QD705, and for decanal + QD705. While signal was seen under the Total Light filter set for all three conditions, blue-specific signal was found only when the *Listeria* innocua were present. Further, QD705-specific signal was seen whenever the QD705 were mixed with the decanal, even in the absence of the *Listeria* innocua. It is this final observation that led to the discovery of Enzyme-independent Photon Emission (EiPE). The location of the QD705 was validated by epifluorescence.

### Example 3: Wavelength-Specific Signal Generation Using EiPE with Various QDs.

Four different solutions were prepared and dispensed into individual eppendorf tubes. The first tubed contained 80 µl 1x PBS and 20 µl of coelenterazine-h. The remaining tubes contained 75 µL of 1x PBS, 20 µL of coelenterazine-h, and 5 µL of QD655, QD705, or QD800. Upon mixing, the tubes were placed into the IVIS100 and visualized under the Total Light, 530-550nm, 610-630nm, 575-650nm, 695-770nm, and 810-870nm emission filters, with the exposure time set to 60 seconds. The location of the various QDs was verified using epifluorescence. The resulting photon flux (photons/second/cm²; p/s/cm²) from the enzyme-independent luminescence was normalized to tube 1, which was the PBS control.

Wavelength-specific signal was observed for each solution that contained the QDs, with the maximum emission observed under the correctly corresponding emission filter. The QD location was verified using epifluorescence. The signal of each solution was compared to the substrate control, and a massive increase in signal was observed (Figure 3). This increase approached nearly 300-fold when the QD705 and QD800 were used, indicating the massive specificity of the signal.

### Example 4: Wavelength-Specific Signal Generation Using EiPE with Various Fluorophore-Embedded Polystyrene Microspheres.

Five different solutions were prepared and dispensed into individual wells of a black 96 well plate. The first four contained 70 µL of 1x PBS, 20 µL of coelenterazine-h, and 10 µL of fluorescent polystyrene microspheres with the noted excitation/emission maxima (505nm/550nm, 580nm/605nm, 660nm/680nm, and 488/650nm). The fifth well contained 80 µL of 1x PBS and 20 µL of coelenterazine-h. The plate was placed into the IVIS100 and visualized under the Total Light, 470-490nm, 530-550nm, 610 Long Pass, 610-630nm and 695-770nm emission filters, using an exposure time of 60 seconds.

Wavelength-specific signal was observed for each solution that contained the polystyrene microspheres, with the maximum emission observed under the correctly corresponding emission filter (Figure 4A to 4F).

### Example 5: The Effect of Different Substrates on Light Generation Using EiPE

Different derivatives of coelenterazine (h, natural, hcp, and fcp; SigmaAldrich) were used to investigate the substrate dependence on the observed EiPE effect using two different standard 0.5 µm polystyrene microspheres (LifeTechnologies). The excitation/emission maxima of the microspheres were 580nm/605nm and 505nm/515nm, respectively. Each well contained 70 µL of 1x PBS, 20 µL of the coelenterazine derivative, and 10 µL of the fluorescent microspheres, and distributed into a black 96 well plate accordingly. The plate was then placed into an IVIS100 and visualized under the indicated filter sets using an exposure time of 60 seconds. The location of the microspheres was validated using epifluorescence. For the two types of fluorescent microsphere involved, the resulting EiPE-induced luminescence from each well was normalized to the average photon flux of the "Natural" coelenterazine derivative. The fold increase in signal was plotted versus the coelenterazine derivative used to induce the luminescent response.

As anticipated, the wavelength-specific signal is generated and confirmed to appear under the appropriate emission filters (figure 5). Interestingly, the amount of signal generated varied from each derivative and for each fluorophore used. This indicates that for a given fluorophore used there exists an ideal derivative that promotes maximum signal generation. Similar trends were observed for the 505/550 (A) and 580/605 (B) carboxylate functionalized fluorophore-embedded polystyrene beads (figure 6). The greatest signal enhancement was observed when coelenterazine-h was used, and each derivative generated at least twice as much signal as the control. This result implies that further chemical modifications to the coelenterazine backbone may yield an increased in generated luminescence.

### Example 6: Surface Functionalization Affects the Resulting EiPE-induced Luminescence.

Fluorescent-embedded polystyrene microspheres with different surface modifications (Non-Reactive, Amine, and Streptavidin) were used to investigate the dependence of EiPE on the surface functionalization. Here, Non-Reactive indicates carboxylate functionalized, which is relatively inert in the presence of most chemicals. The wells containing the microspheres consisted of 70 µL of 1x PBS, 20 µL of coelenterazine-h, and 10 µL of the respective microsphere. The fourth well contained 80 µL of 1x PBS and 20 µL of coelenterazine-h. Finally, another control was established using non-fluorescent carboxylate microspheres following the standard protocol of 70 µL of 1x PBS, 20 µL of coelenterazine-h, and 10 µL of the respective microsphere.

Solutions containing identical concentrations of the microspheres and Coelenterazine-hprepared in a black 96-well plate were observed under the Total Light filter set of an IVIS100 bioluminescence imaging system. The resulting photons/second/cm² (p/s/cm²) flux is shown in Table II.

**Table II: Surface functionalization affects the resulting EiPE-induced luminescence**

| **Surface** | **p/s/cm²/(x10⁶)** |
|---|---|
| Non-Reactive | 2.13 |
| Amine | 1.63 |
| Streptavidin | 3.45 |
| Control | 0.16 |
| Blank µSpheres | 1.02 |

As can be seen (Table II) there exists a dependence on the surface functionalization.

### Example 7: Changing the Surface Area of ReSyn® Beads and its Effect on EiPE Signal Generation.

Non-fluorescent polymeric microspheres with varying degrees of crosslinking provided by ReSyn® were used to investigate the surface area dependence of EiP

E. A ReSyn® bead consists of a long polymeric chain that freely and naturally winds around itself generating a microsphere with a massive surface area to volume ratio. As such, the polymer generates a bead-like structure that is extremely porous and allows the free movement of liquids throughout its entirety. ReSyn® beads were fabricated such that they had varying degrees of crosslinking, thus regulating the amount of surface area available for the EiPE reaction. Four types of microspheres were used: A, B, C, and M, where the degree of cross-linking increased, and the available surface area decreased, from A to B to C. The type M was a magnetized bead with an unspecified degree of crosslinking, but was anticipated to be similar to A. In triplicate, solutions containing 70 µL of 1x PBS, 20 µL of coelenterazine-h, and 10 µL of the respective microspheres were distributed into individual wells of a black 96 well plate. As a control, a solution consisting of 80 µL of 1x PBS and 20 µL of coelenterazine-h was prepared and dispensed in triplicate into individual wells of a second black 96 well plate. The two plates were then placed into an IVIS Spectrum and observed under all the available filter sets 2 (20nm band pass filters which encapsulate the emission from 490nm to 850nm) using an exposure time of 30 seconds. The average standard deviation of the photon flux (p/s/cm²) found for each type of bead and the control was then calculated and plotted against the central wavelength of the respective band pass filter.

As is indicated (figure 7), ReSyn® A had the least amount of crosslinking, followed by ReSyn® B and then ReSyn® C. ReSyn® M is a magnetic variant of ReSyn® A, whereas Coel indicates a solution where no beads are present but only the substrate in 1x PBS. While the resulting emission maxima for each ReSyn® bead occurred at the same region (510-530nm), the intensity of the emission is strongly correlated to the degree of crosslinking. The results indicate that more signal is generated if more surface area is available, suggesting that the surface of the bead strongly influences the EiPE effect

### Example 8: Wavelength-Specific Signal Generation Using EiPE with QD705-Labelled ReSyn® Beads

Custom non-fluorescent polymeric microspheres labeled with varying concentrations of QD705 were provided by ReSyn® and used to demonstrate the ability to create wavelength-dependent luminescent particles. The microspheres were labeled with four different concentrations of QD705: 0, 0.037, 0.081, and 0.152 µM. In triplicate, solutions containing 70 µL of 1x PBS, 20 µL of coelenterazine-h, and 10 µL of the microspheres were prepared and dispensed into individual wells of a black 96 well plate. The plate was then placed into an IVIS Spectrum and observed under the 710 nm - 730 nm filter set. The resulting photon flux was normalized by the average signal observed from the highest QD705 concentration.

As can be seen from the plotted data (figure 8), the most intense QD705 signal occurred in the case where the concentration was at the highest. The resulting luminescence decreased in a linear manner. This indicates that there exists the possibility to create biocompatible polymeric backbones to which a fluorophore or a fluorescent nanoparticle may be attached, and the wavelength of the luminescence predicted.

### Example 9: Wavelength Specific Signal Generation Using EiPE with Rhodamine6-Loaded Lipid Nanoparticles (LNPs) from Global Acorn®.

Custom lipid nanoparticles (Global Acorn, UK) that contained no fluorophore (C LNP), were surface labeled and contained rhodamine-6 (R1), or that only contained rhodamine-6 (R2), were used to further demonstrate the ability to create wavelength-dependent luminescent particles that are highly biocompatible. Three different solutions consisting of 70 µL of 1x PBS, 20 µL of coelenterazine-h, and 10 µL of the respective lipid nanoparticles were prepared and dispensed into individual wells of a black 96 well plate. The plate was placed into an IVIS100 and visualized under the indicated filter sets at an exposure time of 60 seconds. The resulting photon flux (p/s/cm²) for each particle type were normalized to the value found for the C LNP.

As can be seen (figure 9), the LNP that was both labeled with and contained the rhodamine-6 produced the greatest amount of signal under the Total Light, 575-650nm, and 610LP emission filters. This demonstrates that there exists the possibility to create biocompatible polymeric backbones to which a fluorophore or a fluorescent nanoparticle may be attached, and the wavelength of the luminescence predicted.

### Example 10: Wavelength-Specific Signal Generation Using EiPE with Fluorophore-Embedded Microspheres in the Presence of Fixed J774A.1 Murine Macrophages in Suspension.

J774A.1 murine macrophages were grown in standard DMEM medium with 10 % fetal bovine serum. Starting from a fresh culture at approximately 70% confluence, the cell culture medium was removed and replaced with 5 mL of Trypsin. The cells were exposed for 5 minutes at 37 °C. The resulting cell suspension was then placed into a 15 mL Falcon tube and centrifuged for 5 minutes at 1000 rpm. The cells were washed 3 times using 1x PBS before being fixed in suspension using 1% PFA for 15 minutes. Upon completion, the fixed cells were again washed 3 times in 1x PBS before being counted. The cell concentration was then adjusted such that a concentration of 70,000 cells per 100 µL was achieved. Subsequently, 100 µL of the cell suspension was aliquoted into 36 wells of a black 96 well plate.

Red fluorescent polystyrene microspheres (0.5 µm diameter) with an excitation maximum at 580 nm and an emission maximum of 605 nm, with an initial concentration of 2 % solids (Life Technologies), were serially diluted by 12 two-fold increments using 1x PBS. From the dilutions, 10 µL were added to each well with each dilution being added to three individual wells to create triplicates. As such, a bead to cell ratio ranging from 20,788 beads:cell down to 20 beads:cell was achieved. Further, 20 µL of coelenterazine-h was added to each well. The completed plate was then placed into an IVIS Spectrum and visualized under all the available filter sets (ranging from 490nm - 850nm) using an exposure time of 60 seconds. The resulting average and standard deviation of the total photon flux (p/s/cm²) for each well under each filter was plotted versus wavelength. Each condition was repeated in triplicate.

As can be seen (figure 10), a substantial wavelength-specific signal was generated whenever the beads were present, with the magnitude of the signal dependent upon the microsphere-to-macrophage ratio. The microsphere-specific signal is clearly discernable from the background at as few as 20 microspheres per macrophage (inset). This ratio is generally quite achievable suggesting that targeted EiPE could be developed for specific *in vitro* and *in vivo* applications.

### Example 11: The Existence of EiPE under in vivo Conditions.

Two solutions consisting of either 50 µL of coelenterazine-h with 150 µL of 1x PBS or 50 µL of coelenterazine-h, 50 µL of QD705, and 100 µL of 1x PBS were prepared in eppendorf tubes and used to fill two different syringes. The dorsal side of four 6-week old female BALB/c mice was razed and the mice placed under general anesthesia (isofluorane) before being injected with either 50 µL of the control (no QD705, 1 mouse) or 50 µL of the solution containing the QD705 (3 mice). The mice were then placed into an IVIS100 and kept under anesthesia. They were then immediately visualized under the Total Light, 470 - 490 nm, and 695 - 770 nm filter sets with an exposure time of 60 seconds (figures 11A to 11C). The location of the QD705 was verified using epifluorescence (figure 11D). The total photon flux (p/s/cm²) under each filter set was then plotted (figure 11E).

As can be seen (figure 11), nearly a three-fold increase in total signal is observed when the Coel + QD705 solution is present compared to the Coel control. This contrast is even stronger when the QD705-specific filter is used, providing a nearly 20-fold increase in signal. Also acquired was one mouse that received a QD705 injection without the presence of the coelenterazine-h substrate. As anticipated minimal signal was observed in this case. The results shown here demonstrate the potential of EiPE as a tool to create *in vivo* luminescence generation. Given the previous evidence verifying the use of biocompatible markers, the EiPE phenomenon contains significant potential for *in vivo* and *in vitro* uses with the ability to generate wavelength-specific signals.

### Example 12: Using EiPE to Generate Fluorophore-Specific Signals from Multiple Luminescent Sources Covering Nearly the Entire Optical Spectrum

Coelenterazine-h in 1x PBS was mixed with a multitude of fluorophores and fluorescent nanoparticles, including 505/550nm, 580/605nm, and 660/680nm polystyrene microspheres (Yellow, Pink, and Far Red, respectively); QD655, QD705, and QD800; as well as unbound streptavidin-functionalized Alexa dyes (555, 633, and 700). The solutions were produced in triplicate and distributed into a black 96-well plate. The contents of the wells are defined in the following Table III (please note that each solution was repeated three times though it is only listed once):

**Table III: Contents of the wells**

| | λ_{max em} (nm) | Flourophore^{a} | 1x PBS^{a} | Coelenterazine-h^{a} |
|---|---|---|---|---|
| Yellow µspheres | 515 | 10 | 70 | 20 |
| Alexa555 | 555 | 5 | 75 | 20 |
| Pink µspheres | 605 | 10 | 70 | 20 |
| Alexa633 | 633 | 5 | 75 | 20 |
| QD655 | 655 | 5 | 75 | 20 |
| Far Red µspheres | 680 | 10 | 70 | 20 |
| QD705 | 705 | 5 | 75 | 20 |
| Alexa700 | 700 | 5 | 75 | 20 |
| QD800 | 800 | 5 | 75 | 20 |

| | | | | |
|---|---|---|---|---|
| ^{a}: the values represent the distributed volume in µL | | | | |

The prepared plate was then placed into an IVIS Spectrum and viewed under all available filter sets (ranging from 490nm - 850nm). The observed photon flux for each bead was then normalized to the average photon flux of its maximum emission wavelength. The resulting normalized values were then averaged and the standard deviations calculated, and the data plotted against the filter set used.

As can be seen (figure 12), the appropriate fluorescence emission spectrum of each compound was achieved using EiPE. This demonstrates that EiPE can be applied to a wide range of fluorophores nearly regardless of their emission wavelength. Furthermore, EiPE can be used to determine spectral fingerprints and multiplexing, allowing for unparalleled spectral precision.

### Example 13: Multiplexed spectral deconvolution using EiPE.

Polystyrene beads doped with one of three fluorophores (A,B,C) were mixed v/v (AB,AC,BC,ABC) in a 96 well plate in the presence of Coelenterazine-h and treated to reveal EiPE light. Multi-well plate was visualized first using fluorescence illumination (figure 13; *left panels*) as a control, and next using EiPE light generation (figure 13; *right panels*). Spectral deconvolution was performed using 8filter sets covering the visual spectrum range of 490 to 730 nm with 20nm bandwidths. The three "component" channels Spectral deconvolution revealed the presence of the beads both alone and mixed together identically for fluorescence and EiPE illumination thereby validating the utility of the latter as a novel alternative luminescence based method for multiplexed spectral deconvolution of mixed fluorophores.

## Claims

1. A method for generating a luminescent signal, comprising the step of contacting a luciferin or reactive intermediate thereof with at least one physical surface in the absence of a luciferase, wherein the interaction of said luciferin with said physical surface generates a detectable luminescent signal.

2. The method according to claim 1, wherein said physical surface is the surface of a particle or biological matrix.

3. The method according to claim 2, wherein said particle is a metal, lipid, resin, and/or polymer microsphere or nanoparticle.

4. The method according to claim 2, wherein said biological matrix is chosen from actin matrix, collagen matrix, microtubule, microfilament and biofilm.

5. The method according to any one of claims 1 to 4, wherein said luciferin is chosen from decanal, coelenterazine and coelenterazine analogs.

6. The method according to any one of claims 1 to 5, wherein said physical surface comprises a fluorescent molecule, and wherein said detectable luminescent signal is emitted by said fluorescent molecule.

7. The method according to claim 6, wherein said physical surface is the surface of a fluorescent particle chosen from quantum dot, fluorescent polystyrene microsphere and fluorescent lipid nanoparticle.

8. The method according to any one of claims 1 to 7, wherein said physical surface is coated with a molecular probe.

9. The method according to any one of claims 6 to 8, wherein said luciferin is contacted with different fluorescent molecules, preferably covering the optical and near infrared spectrum, each fluorescent molecule being bound to a separate physical surface.

10. The method according to any one of claim 1 to 9, wherein said luminescent signal is for imaging or sensing a biological target, *in vitro* or *in vivo.*

11. A kit for performing the method according to any one of claims 1 to 10, comprising:
- at least one luciferin as defined in claim 1 or 5 ,
- at least one physical surface as defined in any one of claims 1 to 4 and 6 to 9, and
- instructions for the performance of the method according to any one of claims 1 to 10, and wherein the kit does not comprise any luciferase.

12. The kit according to claim 11, wherein the physical surface comprises a fluorescent molecule.

13. Use of a luciferin as defined in claim 1 or 5, in the presence of at least one physical surface as defined in any one of claims 1 to 4 and 6 to 9 and in the absence of a luciferase, to produce a detectable luminescent signal.

14. The use according to claim 13, wherein said physical surface comprises a fluorescent molecule and said detectable luminescent signal is produced by the fluorescent molecule.
